# EUROPEAN PATENT SPECIFICATION

(11) **EP 1 727 910 B1**
(45) Date of publication and mention of the grant of the patent: **07.10.2009**
(21) Application number: 05721068.4
(22) Date of filing: 14.03.2005
(51) Int. Cl.: C12Q 1/68

(54) **METHOD OF DETECTING NUCLEIC ACID USING AMPLIFICATION ON AN ARRAY**
VERFAHREN ZUM NACHWEIS VON NUKLEINSÄUREN DURCH AMPLIFIKATION AUF EINEM ARRAY
PROCEDE DE DETECTION D'ACIDE NUCLEIQUE EN UTILISANT L'AMPLIFICATION SUR MICROPLAQUETTE

(30) Priority: 12.03.2004 JP 2004070986
(43) Date of publication of application: 06.12.2006
(73) Proprietor: CANON KABUSHIKI KAISHA, Ohta-ku Tokyo 146-8501 (JP)
(72) Inventor: OKAMOTO, Tadashi; c/o CANON KABUSHIKI KAISHA, Ohta-ku, Tokyo 1468501 (JP)
(74) Representative: TBK-Patent
(86) International application number: PCT/JP2005/004881
(87) International publication number: WO 2005/087950

(56) References cited:
- WO-A-01/48242
- WO-A-01/71041
- WO-A-02/081743
- WO-A-03/019189
- WO-A-20/04017068
- PATENT ABSTRACTS OF JAPAN vol. 2002, no. 02, 2 April 2002 (2002-04-02) & JP 2001 299346 A (NABA HIROYUKI), 30 October 2001 (2001-10-30)

### SEQUENCE LISTING

<110> Canon Kabushiki Kaisha
<120> Method of detecting nucleic acid
<130> 10009684W001
<150> JP2004-070986
   <151> 2004-03-12
<160> 19
<170> Patentin version 3.3
<210>
   <211> 22
   <212> DNA
   <213> Synthesized
<400> 1
   ctcttgccat cggatgtgcc ca 22
<210> 2
   <211> 26
   <212> DNA
   <213> Synthesized
<400> 2
   atacctttgc tcattgacgt tacccg 26
<210> 3
   <211> 24
   <212> DNA
   <213> Synthesized
<400> 3
   tttgctcatt gacgttaccc gcag 24
<210> 4
   <211> 24
   <212> DNA
   <213> Synthesized
<400> 4
   actggcaagc ttgagtctcg taga 24
<210> 5
   <211> 23
   <212> DNA
   <213> Synthesized
<400> 5
   atacaaagag aagcgacctc gcg 23
<210> 6
   <211> 25
   <212> DNA
   <213> Synthesized
<400> 6
   cggacctcat aaagtgcgtc gtagt 25
<210> 7
   <211> 26
   <212> DNA
   <213> Synthesized
<400> 7
   gcggggagga agggagtaaa gttaat 26
<210> 8
   <211> 22
   <212> DNA
   <213> Synthesized
<400> 8
   atccaaccgc aggttcccct ac 22
<210> 9
   <211> 23
   <212> DNA
   <213> Synthesized
<400> 9
   gcggcaggcc taacacatgc aag 23
<210> 10
   <211> 24
   <212> DNA
   <213> Synthesized
<400> 10
   tgagggagaa agtgggggat cttc 24
<210> 11
   <211> 25
   <212> DNA
   <213> Synthesized
<400> 11
   tcagatgagc ctaggtcgga ttagc 25
<210> 12
   <211> 24
   <212> DNA
   <213> Synthesized
<400> 12
   gagctagagt acggtagagg gtgg 24
<210> 13
   <211> 25
   <212> DNA
   <213> Synthesized
<400> 13
   gtacggtaga gggtggtgga atttc 25
<210> 14
   <211> 24
   <212> DNA
   <213> Synthesized
<400> 14
   gaccacctgg actgatactg acac 24
<210> 15
   <211> 25
   <212> DNA
   <213> Synthesized
<400> 15
   tggccttgac atgctgagaa ctttc 25
<210> 16
   <211> 23
   <212> DNA
   <213> Synthesized
<400> 16
   ttagttacca gcacctcggg tgg 23
<210> 17
   <211> 22
   <212> DNA
   <213> Synthesized
<400> 17
   tagtctaacc gcaaggggga cg 22
<210> 18
   <211> 22
   <212> DNA
   <213> Synthesized
<400> 18
   atccagccgc aggttcccct ac 22
<210> 19
   23
   <212> DNA
   <213> Synthesized
<400> 19
   gcggcaggct taacacatgc aag 23

## Description

### TECHNICAL FIELD

The present invention relates to a method of detecting a nucleic acid, and more particularly to a method of detecting a nucleic acid using a primer array and a PCR method.

### BACKGROUND ART

Base sequence analysis of genome or the like of various living beings including human beings has intensively proceeded, with the result that gene analysis has come into use for diagnosis of a genetic disease, cancer, infectious disease, lifestyle-related disease, or the like; decision of a therapeutic strategy; check after treatment; and prognostic expectation. Moreover, in another field, gene analysis is beginning to be used for distribution management of foods such as meat and grain.

Among techniques used for the gene analysis, a nucleic acid array immobilized with plural nucleic acids such as DNA probes, oligonucleotide probes, and cDNA is one of the techniques that has particularly drawn attention since the late 1990s. The nucleic acid array may also be referred to as DNA array, oligonucleotide array, cDNA array, or the like, and in some situations, as nucleic acid chip or DNA chip. Gene analysis techniques using a nucleic acid array or a nucleic acid array itself essentially have an advantage that multiple and various solid-phase hybridizations can be performed simultaneously, although which is not described in detail herein because those techniques have been generally well known recently.

On the other hand, the PCR (polymerase chain reaction) method that has been considered as an important technique for gene analysis does not go out of fashion today and is one technique most widely used in the gene analysis.

Since the PCR method was devised, some improvements and evolutions have been accomplished. For example, the traditional PCR method was performed for one gene or one base sequence in the form that a base sequence to be amplified was sandwiched by two primers. However, recently, PCR is performed using two primers sandwiching base sequences to be amplified that include different base sequences of plural genes or plural base sequences. That is, amplification of plural genes or the like can be realized using only two (common) primers. Such PCR method is generally referred to as a universal PCR method. For example, when plural infecting organisms causing infectious diseases are to be identified, PCR is performed using primers which sandwich base sequences including a part of base sequences of a specific rRNA (for example, 16s rRNA) unique to the respective infecting organisms and have common base sequences in the respective infecting organisms, and the aforementioned unique base sequences are detected, to thereby identify and detect the respective infecting organisms. Such method has an advantage that PCR procedures are not required for the respective plural genes or the like.

On the other hand, a procedure called multiplex PCR has been developed. This procedure is intended to amplify plural genes or the like with different primer sets in one batch. Although this procedure has a difficulty in design, setting of a primer strand length, base sequence, and PCR conditions or the like, basically, plural and multiple PCR procedures can be performed simultaneously. Moreover, in some situations, improved quantitative detection can be expected through the reactions in the same batch.

Further, a procedure that is a combination of the above-described chip technique and PCR method has also been proposed.

There has been disclosed one procedure of so-called solid phase PCR that is used for performing plural PCR procedures on one solid-phase by immobilizing a set of PCR primers including two oligonucleotides on a matrix in a nucleic acid array (see Japanese Patent Application Laid-Open No. 2001-299346). According to such a procedure, two amplified products that have been amplified on the matrix form an inverted U-shaped hybridized product mutually by keeping the products under hybridization conditions together with a nucleic acid array after PCR. A gene to be detected can be identified and detected by detecting the hybridized product using, for example, a fluorescent intercalator dye.

Moreover, a solid-phase PCR method that is performed on a microplate having plural wells has been proposed (see THCH NOTE Vol. 3, No. 16, published by Nalge Nunc International). That is, the method of PCR in which, one primer of a set of PCR primers is covalently immobilized on the well surface of the microplate, and a template nucleic acid and both primers are used in the wells for PCR. Detection is performed separately using a detection probe for PCR amplified products that are elongated from the primer immobilized on the well surfaces.

Conventional techniques relating to the present invention, that is, gene detection techniques, nucleic acid array techniques, PCR methods, and solid-phase PCR methods have been outlined above.

The aforementioned solid-phase PCR is basically used for one PCR reaction on one matrix or one well. and it is difficult to be applied to the above-described universal PCR or multiplex PCR. Moreover, in the method described in Japanese Patent Application Laid-Open No. 2001-299346, hybridization is sterically limited because an inverted U-shaped hybridized product is finally formed in a microregion. Moreover, because the reactions of the aforementioned method described in TECH NOTE Vol. 3, No. 16 are performed in microplate wells, the numbers of the reactions are limited compared to reactions in the case of a nucleic acid array, and a large volume of a reaction solution is required, so that the detection efficiency may need more improvement. Further, the detection in those techniques requires another step after amplification, which is performed using, for example, a fluorescent intercalator or another detection probe.

Moreover, Japanese Patent Application Laid-Open No. 2003-523183 discloses an array for detecting and comparing expression patterns of plural target polynucleotides from at least two different biological sources, which includes at least two groups of oligonucleotide primers immobilized on a discrete region of a solid-phase support. Each group of oligonucleotide primers is selected for a specific target polynucleotide and includes a sequence complementary to the sequence of the specific target polynucleotide. In the array, each group can be identified by the position on the solid-phase support. Further, the array can be used for detecting expression patterns of target polynucleotides from single biological source.

However, in order to detect different genes, only one set of primers is prepared for each detection. In the case where different microorganisms each having extremely similar sequence, for example, infecting organisms of infectious diseases are identified, determination may not be performed.

WO 01/71041 A2 discloses a microarray-based analysis of polynucleotide sequence variations using immobilized primers on a microarray and performing PCR. During the PCR reaction, a double stranded target polynucleotide is denaturized into single strands. In one embodiment immobilized primers can be used together with primers in the reaction solution, which are complementary to the 3'-end of the nascent immobilized strands. After PCR, the amplified and optionally labeled target polynucleotide is detected by using standard methods including the incorporation of fluorescent labels into the newly synthesized strands.

WO 01/48242 A2 is directed to methods for amplifying and detecting multiple polynucleotides on a solid phase support by using immobilized primers and conducting PCR. According to one embodiment, target genes are detected by using immobilized specific 5'-end primers and solution phase 3'-end primers. Furthermore, the target nucleic acid is detected by using nucleotide labeled monomers.

WO 02/081743 describes a polynucleotide analysis using a combinatorial PCR, which comprises a two-step process using multiple primer immobilized on solid supports. In a first step, PCR is conducted in the presence of multiple primers in the solution, which may be identical or different to the immobilized primers. In a second step, the resulting product of the PCR is detected by hybrid formation or by second strand synthesis using labeled nucleotide monomers.

### DISCLOSURE OF THE INVENTION

In view of the above-described circumstances, the present invention relates to a solid-phase PCR method which enables simple, high-efficiency, and high-precise detection of various genes, and an object of the present invention is to provide a method of detecting a nucleic acid which may be widely utilized in fields on the basis of gene detection such as diagnosis, treatment, or prognostic expectation in the medical field; or distribution management in the food field.

The aforementioned object is accomplished by the following aspects of the present invention.

More specifically, a first aspect of the present invention relates to a method as defined in claim 1.

The aforementioned method enables the universal PCR on a nucleic acid array.

A second aspect of the present invention relates to a method as defined in claim 2.

The above-described methods enable the solid-phase universal PCR, solid-phase multiplex PCR and solid-phase universal multiplex PCR using the nucleic acid array, which have not been accomplished by conventional techniques.

In the aforementioned detection method, each A-strand may include one or more of the plural base sequences to be detected.

One feature of the second aspect of the present invention is to utilize a labeled monomer as a substitute for a part or all of at least one of nucleotide monomers used in PCR reactions in order to simplify a detection step. The method according to the aspect enables the solid-phase PCR capable of performing detection immediately after PCR, solid-phase universal PCR capable of performing detection immediately after PCR, solid-phase multiplex PCR, or solid-phase universal multiplex PCR without performing two-tiered steps in which hybridization is performed after PCR.

Further, the method according of the first and second aspect of the present invention is characterized in that a nucleic acid having the same base sequence as one selected from a region nearer the 5'-end of A-strand than a base sequence to be detected which is nearest the 5'-end (A-strand elongating primer) is added to the B-strand elongating primer, and the nucleic acid is used as a primer which is not immobilized on a substrate, so that the amplifying efficiency of a target base sequence in PCR can be improved.

Moreover, in a further aspect of the method according to the first and second aspect of the present invention, the method is characterized in that PCR reactions and detection are performed in the form in which primer arrays are present in the same container. Further, the PCR reactions and detection can be performed while being observed continuously using the same means.

Note that reference symbol "n" in the methods according to the respective aspects represents the same number (integer).

The present invention enables the solid-phase universal PCR, solid-phase multiplex PCR, or solid-phase universal multiplex PCR using nucleic acid arrays. Moreover, the present invention enables, by using a labeled nucleotide monomer in PCR, the solid-phase PCR capable of performing detection immediately after PCR, solid-phase universal PCR capable of performing detection immediately after PCR, solid-phase multiplex PCR, or solid-phase universal multiplex PCR.

That is, according to the present invention, simple, high-efficiency, and high-precise detection of various and multiple genes can be performed.

Other features and advantages of the present invention will be apparent from the following description taken in conjunction with the accompanying drawings, in which like reference characters designate the same or similar parts throughout the figures thereof.

### BRIEF DESCRIPTION OF THE DRAWINGS

Fig. 1 is a schematic view for illustrating the solid-phase universal PCR according to the present invention; and
Fig. 2 is a schematic view for illustrating the solid-phase universal PCR and incorporated label according to the present invention.
Fig. 3 schematically illustrates an apparatus for carrying out the method according to the invention.
Fig. 4 schematically illustrates another apparatus for carrying out the method according to the invention.
Fig. 5 schematically illustrates still another apparatus for carrying out the method according to the invention.

### BEST MODE FOR CARRYING OUT THE INVENTION

Preferred embodiments of the present invention will now be described in detail in accordance with the accompanying drawings.

A method of detecting a nucleic acid according to a first mode of the present invention includes the aforementioned steps (1) to (6). This method enables the universal PCR on a primer array.

In this case, if the base sequence to be detected is, for example, of a gene having a double-stranded nucleic acid, the sense base sequence is basically the same as the anti-sense base sequence. It is obvious that analysis of the anti-sense sequence reveals the sense base sequence complementary to the sequence (that is, the base sequence of the gene).

Each operation in each step can be performed using the known method. In the detection step as the final step, any procedure which is capable of detecting a formed hybridized product may be used. For example, a detection method using a fluorescent intercalator dye or fluorescent groove binder dye which interacts with a double-stranded nucleic acid to emit or enhance fluorescence may be utilized. Alternatively, there may also be adopted a procedure for labeling a nucleic acid having a base sequence complementary to a base sequence located in the position nearer 3'-end than that of the 3'-end of the base sequence to be detected of A-strand, which is used as a primer in the step (4) (which is a B-strand elongating primer and a primer derived from a segment nearer the 3'-end of A-strand; herein, the phrase "derived from a position nearer the 3'-end" means "derived from a segment located in the position nearer 3'-end than that of the base sequence to be detected which is nearest the 3'-end"), with a fluorescent dye such as fluorescein, tetramethylrhodamine, Cy3, or Cy5, or radioisotope. Note that, in the case of using the fluorescent dye, a fluorescent microscope may be used for observation, and a step of observing a label using, for example, a confocal fluorescent microscope may further be included.

In this method, one primer of a set of primers to be used PCR reactions is immobilized on a solid-phase, and the other (B-strand elongating primer) is dissolved in a reaction solution before use. The B-strand elongating primer has functions as a common primer for amplifying each base sequence to be detected. Further, the steps (2) and (3) of the first mode are as follows:
(2) preparing as primers, nucleic acids each having one of the plural base sequences to be detected, immobilizing the respective primers independently in separate regions on a substrate, and preparing a primer array in which the respective base sequences to be detected are distributed in the primer-immobilized regions; and
(3) preparing a nucleic acid having a partial and sequential base sequence within the region between a 5'-end of the A-strand and the base sequence to be detected which is located nearest the 5'-end as a primer for elongating the A-strand and preparing a nucleic acid having a base sequence complementary to a partial and sequential base sequence within the region between a 3'-end of the A-strand and the base sequence to be detected which is located nearest the 3'-end as a primer for elongating the B-strand.

That is, in addition to the B-strand elongating primer, a nucleic acid having the same base sequence as a sequential and partial base sequence which is located in the position nearer 5'-end than that of the 5'-end of the base sequence to be detected of A-strand (which is an A-strand elongating primer and a primer derived from a segment nearer the 5'-end of A-strand: herein, the phrase "derived from a position nearer the 5'-end" means "derived from a segment located in the position nearer 5'-end than that of the base sequence to be detected which is nearest the 5'-end") may be dissolved in a reaction solution to be used concomitantly as the second common primer. In some situations, the amplifying efficiency in such concomitant use may be higher.

Note that, in some situations, this method may include a step of removing a substance other than the aforementioned hybridized product (such as a template nucleic acid, or a nucleotide monomer or enzyme used for PCR reactions) through a washing operation for removing a reaction solution on the substrate.

Those methods relate to the universal PCR as described above, and the method of detecting a nucleic acid according to a second mode of the present invention includes the above-described steps (1) to (6). This method enables the multiplex PCR.

In this case, the same detection method as that described above may be used for detecting a hybridized product. The aforementioned detection method may be used for plural single-stranded nucleic acids each having one or more partial and sequential base sequences to be detected (A-strand group: A1-strand to An-strand: n ≥ 2). Moreover, the steps (2) and (3) according to the second mode may be changed as follows:
(2) preparing nucleic acids as primers each having one of the plural base sequences to be detected, immobilizing the respective primers independently in separate regions on a substrate, and preparing a primer array in which the respective base sequences to be detected are distributed in the primer-immobilized regions; and
(3) preparing nucleic acids each having a partial and sequential base sequence within the region between a 5'-end of each strand of the A-strand group and the base sequence to be detected which is located nearest the 5'-end as primers for elongating the A-strand (PA-strand group: PA1-strand to PAn-strand: n ≥ 2) and preparing nucleic acids each having a base sequence complementary to a partial and sequential base sequence within the region between a 3'-end of each strand of the A-strand group and the base sequence to be detected which is located nearest the 3'-end as primers for elongating the B-strand (PB-strand group: PB1-strand to PBn-strand: n ≥ 2).

That is, in amplification reactions, plural nucleic acids each having the same base sequence as a sequential and partial base sequence which is located in the position nearer 5'-end than that of the 5'-end of the base sequence to be detected of the aforementioned A-strand group (PA-strand group: PA1-strand to PAn-strand: n ≥ 2) may be dissolved in a reaction solution together with the PB-strand group to be used as common primers. In some situations, the amplification efficiency in this method is higher.

The aforementioned methods enable the solid-phase universal PCR, solid-phase multiplex PCR, or solid-phase universal multiplex PCR using the nucleic acid array.

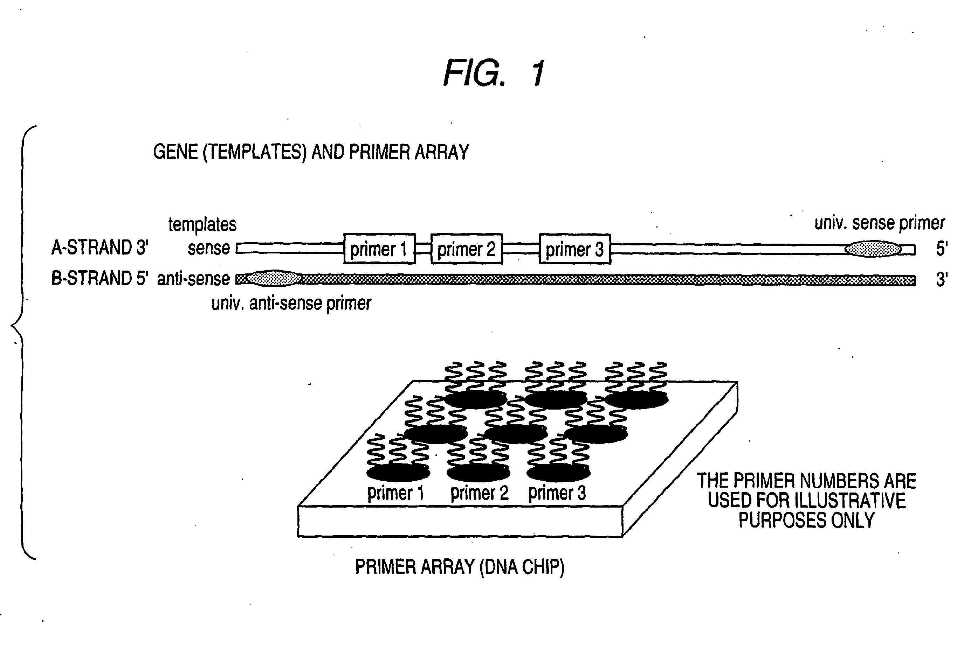

Fig. 1 is a schematic view illustrating the aforementioned method in the case in which A-strand has three base sequences to be detected. In Fig. 1, a primer 1 to a primer 3 are located from the 3'-end to the 5'-end of A-strand, and on B-strand which is complementary to A-strand, a segment is defined as a common primer (univ. anti-sense primer), which is located on the segment having a sequence complementary to that of the segment selected from the region located in the position nearer 3'-end than the 3'-end of the primer 1 which is nearest the 3'-end of A-strand. Moreover, a segment is defined as a common primer (univ. sense primer), which is located on the segment having the same sequence as that in the segment selected from the region located in the position nearer 5'-end than the 5'-end of a primer 3 which is nearest the 5'-end of A-strand. The substrate has regions in which the respective primers having the same sequence as that of segments of the primer 1 to the primer 3 are immobilized, regions in which primers are immobilized are shown in Fig. 1. When PCR reactions are performed on the substrate, amplification and elongation proceed from the primers immobilized on the substrate toward the location predetermined by a common primer. In Fig. 1, at the primer-immobilized region, the 3'-end of the primer fixed at 5'-end on the substrate is elongated and amplified to the position predetermined by a common primer (univ. anti-sense primer) to form a fixed strand corresponding to A-strand, and the fixed strand and a strand corresponding to B-strand which is amplified from B-strand may form a double-strand. By detecting the double-strand, a base sequence to be detected in A-strand can be identified. When a common primer (univ. sense primer) is further added, the amplifying effect of A-strand itself may be expected, in some situations, the amplifying efficiency may be improved.

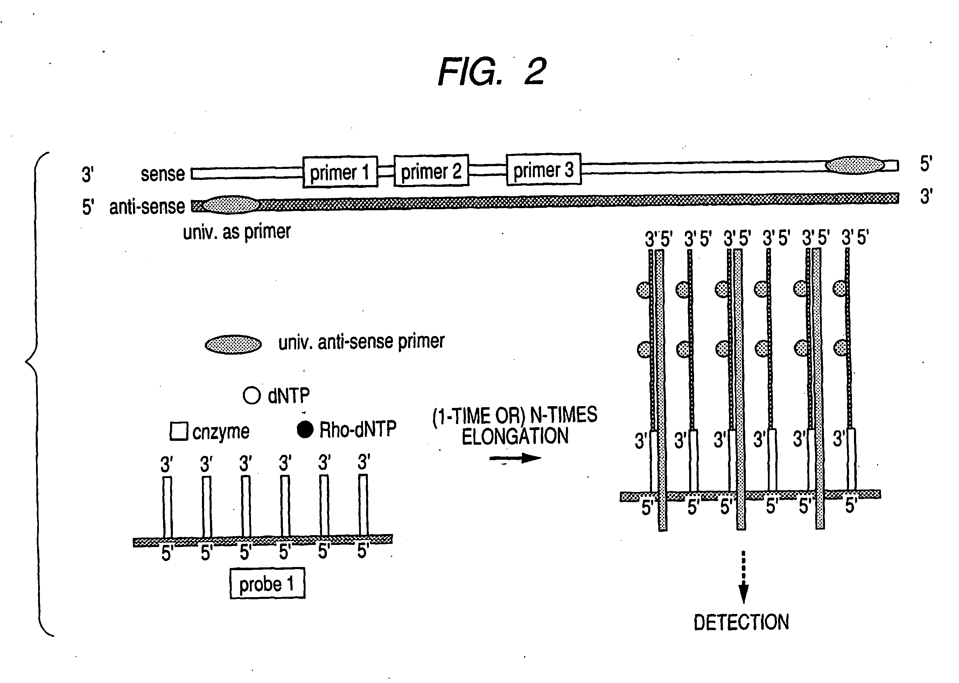

Next, as in the third and forth modes, in order to simplify detection steps, a labeled monomer may be utilized as a substitute for a nucleotide monomer used in PCR reactions. The situation is shown in a schematic view of Fig. 2. That is, when a labeling substance is bound to a part or all of at least one of the nucleotide monomers for elongation which include bases of adenine, guanine, cytosine, and thiamine upon amplification, the aforementioned labeling substance is introduced to a nucleic acid elongated from a primer immobilized on a substrate after amplification. As a result, even if steps subsequent to hybridization in the aforementioned method are omitted, detection can be performed. A labeling substance used in this method is not particularly limited, but includes, for example, the above-described fluorescent dyes and radioisotopes. Also, in the case where a fluorescent dye is used for labeling, in the detection steps in the third and forth modes, an observation step using a confocal fluorescent microscope may be additionally included.

Note that, in some situations, this method may include a step of removing a substance other than a nucleic acid which is elongated and amplified by the PCR reactions and corresponds to A-strand binding to a substrate, for example, a nucleic acid which is elongated and amplified and corresponds to B-strand, a template nucleic acid, a nucleotide monomer used in the PCR reactions, or an enzyme.

This method enables the solid-phase PCR capable of performing detection immediately after PCR, the solid-phase universal PCR capable of performing detection immediately after PCR, the solid-phase multiplex PCR, or the solid-phase universal multiplex PCR without performing two-tiered steps in which hybridization is performed after PCR, and was required to be improved in the aforementioned conventional technique.

The above-described methods are preferred at least in points that PCR reactions and detection are performed in the form in which primer arrays are present in the same container, which is preferred in that an apparatus or the like can be simplified and in that, as described below, PCR reactions and detection can be performed using the same method simultaneously and continuously. In such case, when the PCR reactions and detection are performed using the same method simultaneously and continuously, the process of the PCR amplification can be monitored. Accordingly, this is a preferred mode in that so-called real-time PCR is realized, that is, highly quantitative detection can be performed. An apparatus that is equipped with a PCR reaction container and detection method enabling such a detection method is a preferred mode for carrying out the present invention.

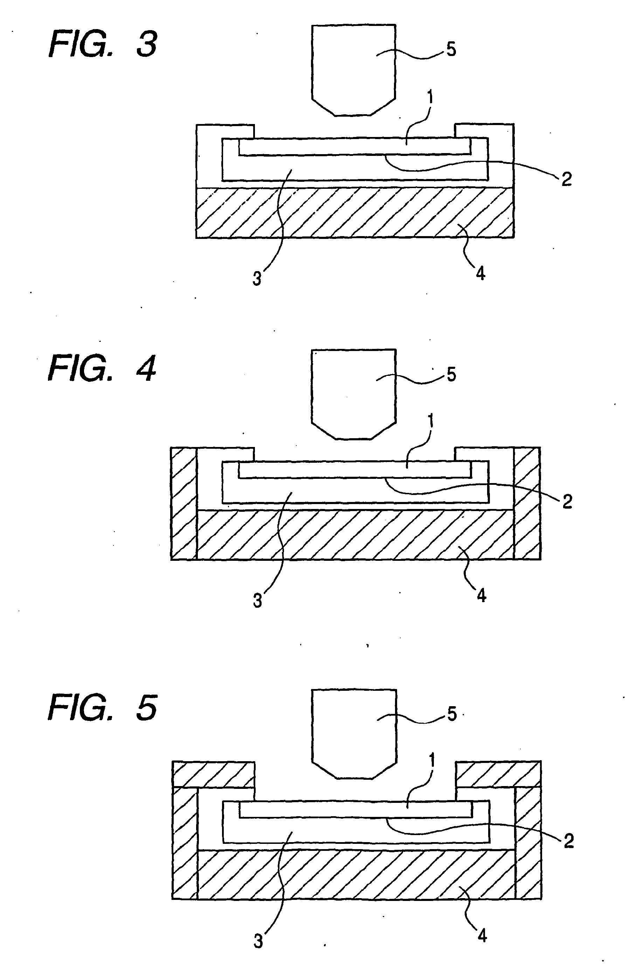

Fig. 3 illustrates such an apparatus. In the apparatus, the PCR container comprises a substrate 1 having a surface 2 with immobilized polymers, a reaction chamber 3 and a temperature controlling unit 4. The substrate is transparent against wavelength used for detection. The reaction chamber is facing to the surface. The temperature controlling unit is placed at a position not preventing operation of detection means 5 which is placed on the side opposite to said surface in relation to said substrate.

The solution mentioned above is introduced from an inlet port (not shown) into the reaction chamber 3 to conduct PCR reactions while the temperature is controlled by the temperature controlling unit 4 in accordance with the aforementioned schedule.

Fig. 4 illustrates another apparatus. In the apparatus of Fig. 4, the temperature controlling unit 4 is arranged such that it surrounds also the lateral surface of the reaction container to increase the contact area.

Fig. 5 illustrates still another apparatus. In the apparatus of Fig. 5, the entire surface except the detection area is covered by the temperature controlling unit 4. Moreover, the present invention describes a kit for detecting a nucleic acid, which includes the aforementioned primer arrays, PCR reaction reagent, and detection reagent. The above-described PCR reaction container may be provided for the kit, and the PCR reaction container may be formed into a cartridge form. Moreover, in the case where a nucleic acid to be detected forms a double-stranded nucleic acid, the detection reagent of the above-described kit for detecting a nucleic acid is preferably a fluorescent dye as an intercalator or a groove binder, which acts on a double-stranded nucleic acid as a fluorescent dye.

### EXAMPLES

Hereinafter, the present invention will be described in detail with reference to examples.

### Example 1 (Solid-phase universal PCR I)

### (1) Preparation of primer

The following seven segments (Ec1 to Ec7) of sense base sequences of genome DNA of 16s ribosomal RNA (rRNA) of Escherischia coli (ATCC#11775) were defined as detection targets of a solid-phase universal PCR, which were selected as primers to be immobilized on solid-phase.
Ec1 5' CTCTTGCCATCGGATGTGCCCA 3' (SEQ ID NO: 1)
Ec2 5' ATACCTTTGCTCATTGACGTTACCCG 3' (SEQ ID NO: 2)
Ec3 5' TTTGCTCATTGACGTTACCCGCAG 3' (SEQ ID NO: 3)
Ec4 5' ACTGGCAAGCTTGAGTCTCGTAGA 3' (SEQ ID NO: 4)
Ec5 5' ATACAAAGAGAAGCGACCTCGCG 3' (SEQ ID NO: 5)
Ec6 5' CGGACCTCATAAAGTGCGTCGTAGT 3' (SEQ ID NO: 6)
Ec7 5' GCGGGGAGGAAGGGAGTAAAGTTAAT 3' (SEQ ID NO: 7).

Those primers were synthesized by a synthesis company (BEX Co., Ltd.) on commission. The 5'-end of each of Ec1 to Ec7 was bound to a thiol (SH) group via a linker for binding to a solid-phase substrate as described below. The Ec1 structure to which a thiol group is bound is shown below as an example.
HS (CH₂)₆OP(O₂) O-dCTCTTGCCATCGGATGTGCCCA

Moreover, the following base sequence (EcAP) was selected as a base sequence of a common primer of the anti-sense sequence:
EcAP 5' ATCCAACCGCAGGTTCCCCTAC 3' (SEQ ID NO: 8)

EcAP was synthesized in a general manner. All the primers were deprotected and purified based on the conventional methods.

### (2) Extraction of Escherischia coli genome DNA

Firstly, an E.coli standard strain was cultured in accordance with the conventional method. 1.0 ml of the microorganism-cultured medium (OD₆₀₀ = 0.7) was collected in a 1.5-ml microtube, and the bacterial cells were collected by centrifugation (8,500 rpm, 5 minutes, 4°C). After the supernatant was discarded, 300 µl of Enzyme Buffer (50 mM Tris-HCl: p.H. 8.0, 25 mM EDTA) was added thereto, and the cells were resuspended using a mixer. The resuspended bacterial suspension was centrifuged again, to thereby collect bacterial cells (8,500 rpm, 5 minutes, 4°C). After the supernatant was discarded, the following enzyme solutions were added to the collected bacterial cells, and the cells were resuspended using a mixer:
Lysozyme 50 µl (20 mg/ml in Enzyme Buffer)
N-Acetylmuramidase SG 50 µl (0.2 mg/ml in Enzyme Buffer).

Next, the resuspended bacterial suspension to which the enzyme solutions were added was left to stand in an incubator at 37°C for 30 minutes, to thereby perform treatment for cell wall lysis. Subsequently, genome DNA was extracted using a kit for purifying a nucleic acid (Mag Extractor. Genome-: manufactured by TOYOBO Co., Ltd.).

Specifically, firstly, 750 µl of a dissolution/adsorption solution and 40 µl of magnetic beads were added to the pretreated microorganism suspension, and the mixture was stirred vigorously for 10 minutes using a tube mixer (Step 1).

The microtube was set on a separatory stand (Magical Trapper) and allowed to stand for 30 seconds, to thereby collect the magnetic particles on the wall surface of the tube, and the supernatant was discarded while the tube was set on the stand (Step 2).

Next, 900 µl of a washing solution was added thereto, and the particles were resuspended by stirring using the mixer for about 5 seconds (Step 3).

Next, the microtube was set on the separatory stand (Magical Trapper) and allowed to stand for 30 seconds, to thereby collect the magnetic particles on the wall surface of the tube, and the supernatant was discarded while the tube was set on the stand (Step 4) .

The procedures of Steps 3 and 4 were repeated, and the second washing was performed (Step 5). Subsequently, 900 µl of 70% ethanol was added thereto, and the particles were resuspended by stirring using the mixer for about 5 seconds (Step 6).

Next, the microtube was set on the separatory stand (Magical Trapper) and allowed to stand for 30 seconds, to thereby collect the magnetic particles on the wall surface of the tube, and the supernatant was discarded while the tube was set on the stand (Step 7).

The procedures of Steps 6 and 7 were repeated, and the second washing was performed with 70% ethanol (Step 8). Subsequently, 100 µl of pure water was added to the collected magnetic particles, and the mixture was stirred for 10 minutes using the tube mixer.

Next, the microtube was set on the separatory stand (Magical Trapper) and allowed to stand for 30 seconds, to thereby collect the magnetic particles on the wall surface of the tube, and the supernatant was collected in a new tube while the tube was set on the stand.

The collected genome DNA of Escherischia coli was subjected to agarose electrophoresis and absorbance measurement with the wavelength of 260/280 nm in accordance with the conventional methods to assay its quality (amount of contaminated low-molecular-weight nucleic acids, the degree of degradation) and collected amount. In this example, about 9 µg of the genome DNA was collected, and degradation of the genome DNA and contamination of rRNA were not observed. The collected genome DNA was dissolved in TE buffer so as to have a final concentration of 50 ng/µl, and the mixture was used in the following examples.

### (3) Manufacture of DNA array

### (3-1) Washing of glass substrate

A glass substrate made of synthetic quartz (size: 25 mm × 75 mm × 1 mm, manufactured by Iiyama Tokusyu Glass Co. Ltd.) was placed in a heat-resistant and alkali-resistant rack and immersed in a washing solution for ultrasonic cleaning which had been adjusted to a predetermined concentration. The substrate was immersed in the washing solution overnight, and ultrasonic cleaning was then performed for 20 minutes. Subsequently, the substrate was taken out and lightly rinsed with pure water, followed by ultrasonic cleaning in ultrapure water for 20 minutes. Next, the substrate was immersed in a 1N sodium hydroxide solution heated to 80°C for 10 minutes. Washing with pure water and washing with ultrapure water were performed again, to thereby prepare a quartz glass substrate for a DNA array.

### (3-2) Surface treatment

A silane coupling agent KBM-603 (manufactured by Shin-Etsu Silicones) was dissolved in pure water so as to have a concentration of 1%, and the mixture was stirred for two hours at room temperature. Subsequently, the glass substrate that had been previously washed was immersed in the aqueous solution of the silane coupling agent and allowed to stand for 20 minutes at room temperature. The glass substrate was drawn up, and its surface was lightly washed with pure water. Subsequently, nitrogen gas was blown on both the sides of the substrate, and the substrate was dried. Next, the treatment with the coupling agent was completed by baking the dried substrate in an oven heated to 120°C for 1 hour, to thereby introduce amino groups to the substrate surface. Next, N-(6-maleimidocaproyloxy)succinimido (hereinafter, abbreviated as EMCS; produced by Dojindo Laboratories) was dissolved in a mixed solvent of dimethylsulfoxide and ethanol (1:1) so as to have a final concentration of 0.3 mg/ml to prepare an EMCS solution. The baked glass substrate was left to cool and immersed in the prepared EMCS solution at room temperature for 2 hours. Through such treatment, the amino groups introduced onto the surface by using the silane coupling agent reacted with succinimido groups of EMCS, to thereby introduce maleimide groups onto the surface of the glass substrate. The glass substrate was drawn up from the EMCS solution, and the substrate was washed with the above-described mixed solvent and further washed with ethanol, followed by drying under nitrogen gas atmosphere.

### (3-3) Primers

The primers to be immobilized on solid-phase (Ec1 to Ec7) described in (1) were separately dissolved in pure water, and each mixture was dispensed so as to have a final concentration (when it was dissolved as described below) of 10 µM, followed by freeze-drying, to thereby remove water.

### (3-4) Discharge of primer DNA by BJ printer, and binding to substrate

An aqueous solution (mixed solvent) containing 7.5 wt% of glycerin, 7.5 wt% of thiodiglycol, 7.5 wt% of urea, and 1.0 wt% of Acetylenol EH (manufactured by Kawaken Fine Chemicals Co., Ltd.) was prepared. Subsequently, each of previously-prepared 7 primers was dissolved in the aforementioned mixed solvent so as to have a prescribed concentration. The resultant DNA solution was filled into an ink tank for a bubble jet printer (trade name: BJF-850, manufactured by Canon Inc.), and the tank was attached to a print head.

Note that the bubble jet printer used herein had been modified so as to enable print on a flat plate. Moreover, the bubble jet printer can perform spotting of about 5 pl of a DNA solution at a pitch of about 120 µm by inputting a printing pattern in accordance with a predetermined file creation method.

Subsequently, a printing operation for one glass substrate was performed using the modified bubble jet printer for preparing a DNA array. After confirming that printing was performed without fail, the array was allowed to stand in a humidified chamber for 30 minutes to react maleimide groups on the surface of the glass substrate with thiol groups at the end of the nucleic acid primer.

### (3-5) Washing

After the reaction was performed for 30 minutes, the DNA solution remaining on the surface was washed out with 10 mM of phosphate buffer (pH.7.0) containing 100 mM of NaCl, to thereby yield a DNA array in which single-stranded DNA was immobilized on the surface of the glass substrate.

### (4) Solid-phase PCR

A method of amplifying Escherischia coli genome DNA was described below.

| | |
|---|---|
| Premix PCR reagent (TAKARA ExTaq) | 25 µl |
| Template Escherischia coli genome DNA | 2 µl (100 ng) |
| Primer EcAP | 2 µl (20 pmole) |
| H₂O | 21 µl |
| Total | 50 µl |

A microchamber in which a primer-binding site of the above-described primer array was covered and the temperature can be controlled was manufactured, and a reaction solution of the aforementioned composition was sealed in the chamber. Then, amplification reactions were performed in accordance with the following protocol. More specifically, after an incubation step at 95°C/10 minutes, a denaturation step at 92°C/45 seconds, an annealing step at 55°C/45 seconds, and an elongation step at 72°C/45 seconds were defined as one cycle, and the cycle was repeated 35 times, and finally an incubation step at 72°C/10 minutes was performed.

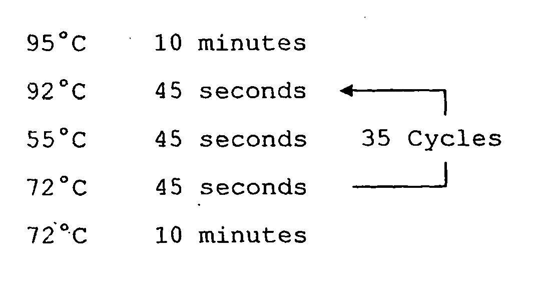

After the completion of the reactions, 1 µl of a solution prepared by previously diluting a dye of which (fluorescence is enhanced under coexistence of a double-stranded nucleic acid, SYBR (registered trademark) Green I (Molecular Probes: trade name SYBR (registered trademark) Green I nucleic acid gel stain 10,000 × concentrate in DMSO) to 200-fold with pure water was added to the chamber, and the primer array was placed under the conditions of 65°C/3 minutes 92°C/2 minutes 45°C/3 hours for hybridization.

Next, the primer array was washed under the conditions of 2 × SSC/0.1% SDS (25°C)/2 minutes - 2 × SSC (20°C)/2 minutes → pure water (10°C)/2 minutes, and the array was taken out of the chamber and dried.

### (5) Fluorescence measurement

The DNA array after the completion of the hybridization reaction was subjected to fluorescence measurement using a fluorescence detecting apparatus for DNA array (manufactured by Axon Instruments, GenePix 4000B) (excitation wavelength: 532 nm, photomultiplier voltage: 400 V). The measurement results are shown in Table 1. Note that, in this example, all the operations are performed twice, so that the results were separately shown in Table 1.

**Table 1**

| Primer No. | Fluorescence luminance | |
|---|---|---|
| | First time | Second time |
| Ec1 | 8,700 | 8,500 |
| Ec2 | 9,100 | 9,050 |
| Ec3 | 7,750 | 7,650 |
| Ec4 | 11,000 | 11,000 |
| Ec5 | 10,400 | 10,550 |
| Ec6 | 7,400 | 7,400 |
| Ec7 | 5,900 | 6,000 |

The numeric values of the fluorescent luminance in Table 1 represent pixel average luminance (resolution: 5 µm). As is clear from Table 1, the results obtained by amplifying genome DNA extracted from Escherischia coli by the solid-phase universal PCR can be detected with high reproducibility.

### Example 2 (Solid-phase universal PCR II)

The solid-phase PCR and fluorescence detection were performed in accordance with the same method as that in Example 1 except that, in addition to the primers used for the solid-phase PCR in Example 1, a common primer (EcSP) of the sense sequence having the following base sequence was used at the same concentration as that of a common primer of the anti-sense sequence.
EcSP 5' GCGGCAGGCCTAACACATGCAAG 3' (SEQ ID NO: 9).

In Example 2, when the solid-phase PCR cycle was repeated 31 times, substantially the same results as those in Example 1 were obtained. The results reveal that the efficiency of the solid-phase PCR could be improved by letting both the common primers of the sense sequence and anti-sense sequence coexist in a solution.

### Example 3 (Solid-phase multiplex universal PCR I)

### (1) Preparation of primers

In this example, genome DNA of rRNA of Pseudomonas aeruginosa (ATCC#10145) will be detected simultaneously with Escherischia coli detected in Example 1. For this purpose, firstly, 8 thiolized primers of the sense sequence for Pseudomonas aeruginosa (Pa1 to Pa8) were synthesized in the same way as that in Example 1. The base sequences of the primers are shown below.
Pa1 5' TGAGGGAGAAAGTGGGGGATCTTC 3' (SEQ ID NO: 10)
Pa2 5' TCAGATGAGCCTAGGTCGGATTAGC 3' (SEQ ID NO: 11)
Pa3 5' GAGCTAGAGTACGGTAGAGGGTGG 3' (SEQ ID NO: 12)
Pa4 5' GTACGGTAGAGGGTGGTGGAATTTC 3' (SEQ ID NO: 13)
Pa5 5' GACCACCTGGACTGATACTGACAC 3' (SEQ ID NO: 14)
Pa6 5' TGGCCTTGACATGCTGAGAACTTTC 3' (SEQ ID NO: 15)
Pa7 5' TTAGTTACCAGCACCTCGGGTGG 3' (SEQ ID NO: 16)
Pa8 5' TAGTCTAACCGCAAGGGGGACG 3' (SEQ ID NO: 17).

Moreover, the following base sequence (PaAP) was selected as a base sequence of a common primer of the anti-sense sequence:
PaAP 5' ATCCAGCCGCAGGTTCCCCTAC 3' (SEQ ID NO: 18).

### (2) Fluorescence detection

Extraction of Pseudomonas aeruginosa genome, manufacture of a DNA microarray immobilized with Escherischia coli primers and Pseudomonas aeruginosa primers, solid-phase PCR (Escherischia coli genome DNA and Pseudomonas aeruginosa genome DNA: 10 ng each, EcAP and PaAP: 20 pmole each), hybridization, washing, etc. were performed in the same way as that in Example 1, and fluorescence detection was performed. The results are shown in Table 2.

**Table 2**

| Primer No. | Fluorescence luminance | |
|---|---|---|
| | Escherischia coli | Pseudomonas aeruginosa |
| Pa1 | 5,800 | 300 |
| Pa2 | 6,050 | 550 |
| Pa3 | 5,150 | 1,700 |
| Pa4 | 7,400 | 650 |
| Pa5 | 6,900 | 1,600 |
| Pa6 | 4,900 | 2,650 |
| Pa7 | 3,900 | 600 |
| Pa8 | - | 350 |

Table 2 shows that all the given primer sequences of rRNA genomes of both Escherischia coli and Pseudomonas aeruginosa were simultaneously detected by the solid-phase multiplex universal PCR.

### Example 4 (Solid-phase multiplex universal PCR II)

The solid-phase PCR and fluorescence detection were performed in the same manner as that in Example 3 except that, in addition to the primers used for the solid-phase PCR in Example 2, common primers of the respective sense sequences of Escherischia coli and Pseudomonas aeruginosa (EcSP and PaSP) were used at the same concentration. The base sequence of PaSP is shown below.
PaSP 5' GCGGCAGGCTTAACACATGCAAG 3' (SEQ ID NO: 19).

In Example 4, under the condition that the cycle of the solid-phase PCR was repeated at given times fewer than those in Example 3 by about one or more, almost the same results as those in Example 3 were obtained. The results reveal that, in the solid-phase multiplex universal PCR, the efficiency of the solid-phase PCR could be improved by letting both the common primers of the sense sequence and anti-sense sequence coexist in a solution.

### Example 5 (Incorporated-label solid-phase universal PCR)

Extraction of Escherischia coli genome, synthesis of primers, and manufacture of a primer array were performed in completely the same manner as that in Example 1, and the solid-phase PCR was then performed using a PCR solution of the following composition.

| | |
|---|---|
| Premix PCR reagent (TAKARA ExTaq) | 25 µl |
| Template Escherischia coli genome DNA | 2 µl (100 ng) |
| Primer EcAP | 2 µl (20 pmole) |
| Cy-3 dUTP (Amersham Biosciences K.K.: 1 mM) | 2 µl (2 nmol) |
| H₂O | 19 µl |
| Total | 50 µl |

Subsequently, the primer array was washed, without keeping the same under to hybridization conditions, under the conditions of 2 × SSC/0.1% SDS (92°C)/2 minutes 2 × SSC/0.1% SDS (92°C)/2 minutes → 2 × SSC/0.1% SDS (25°C)/2 minutes → 2 × SSC (20°C)/2 minutes → pure water (20°C)/2 minutes, and the array was taken out of the chamber and dried.

Subsequently, fluorescence detection was performed in the same manner as that in Example 1. The results are shown in Table 3.

**Table 3**

| Primer No. | Escherischia coli |
|---|---|
| | Fluorescence luminance |
| Ec1 | 11,500 |
| Ec2 | 11,900 |
| Ec3 | 10,000 |
| Ec4 | 14,800 |
| Ec5 | 13,200 |
| Ec6 | 9, 500 |
| Ec7 | 7,700 |

In accordance with the detection method of the present invention, fluorescence detection was performed adopting an incorporated label in the solid-phase universal PCR. As a result, fluorescence detection was easily accomplished as shown in Table 3. Accordingly, the results reveal that fluorescence detection can be performed without requiring the hybridization step.

### Example 6

The solid-phase universal PCR or solid-phase multiplex universal PCR in Examples 2 to 4 was performed using the incorporated label in Example 5. Although the respective fluorescence intensities were different from those in Examples 2 to 4, the results reveal that the rRNA genome DNA of Escherischia coli or the rRNA genome DNAs of both Escherischia coli and Pseudomonas aeruginosa can be detected simultaneously.

### Example 7 (Common primer-labeled solid-phase universal PCR)

Detection of Escherischia coli genome DNA was performed by the solid-phase universal method in completely the same manner as that in Example 1 except that the common primer EcAP was labeled with tetramethylrhodamine, hybridization was performed without coexistence with a fluorescent dye such as SYBR green, and fluorescence detection was performed using the aforementioned tetramethylrhodamine. The structure of tetramethylrhodamine-labeled EcAP is shown below.
5' Rho-CONH(CH₂)₆OP(O₂)O-d ATCCAACCGCAGGTTCCCCTAC 3' (Rho = tetramethylrhodamine).

As a result, almost the same results as those in Example 1 were obtained although the differences in fluorescence intensities were observed.

A method of labeling the common primer of anti-sense sequence was performed in the methods of Examples 2 to 4. As a result, the results confirm that all the methods were effective were obtained.

The present invention is not limited to the above embodiments and various changes and modifications can be made within the spirit and scope of the present invention. Therefore to apprise the public of the scope of the present invention, the following claims are made.

This application claims priority from Japanese Patent Application No. 2004-070986 filed on March 12, 2004, which is hereby incorporated by reference herein.

## Claims

1. A method of detecting a nucleic acid, comprising the steps of:
(1) preparing a single-stranded nucleic acid having plural partial and sequential base sequences to be detected (A-strand) and a single-stranded nucleic acid having a base sequence complementary to a base sequence of the A-strand (B-strand);
(2) preparing nucleic acids as primers each having one of the plural base sequences to be detected, immobilizing the respective primers independently in separate regions on a substrate, and preparing a primer array in which the respective base sequences to be detected are distributed in the primer-immobilized regions;
(3) preparing a nucleic acid having a sequence complementary to a partial and sequential base sequence within the region between a 3'-end of the A-strand and the base sequence to be detected which is located nearest the 3'-end as a primer for elongating the B-strand, and preparing a nucleic acid having a partial and sequential base sequence within the region between a 5'-end of the A-strand and the base sequence to be detected which is located nearest the 5'-end as a primer for elongating the A-strand;
(4) performing PCR reactions using the A-strand and B-strand as templates, and using the primers immobilized on the substrate, the primer for elongating the A-strand, and the primer for elongating the B-strand;
(5) forming a hybridized product of a nucleic acid corresponding to the A-strand which has been elongated and amplified as a result of the PCR reactions and bound to the substrate and a nucleic acid corresponding to the B-strand which has been elongated and amplified and has not bound to the substrate; and
(6) detecting the base sequence to be detected by detecting the hybridized product in the respective primer-immobilized regions in the array.

2. A method of detecting a nucleic acid, comprising the steps of:
(1) preparing a single-stranded nucleic acid having plural partial and sequential base sequences to be detected (A-strand) and a single-stranded nucleic acid having a base sequence complementary to a base sequence of the A-strand (B-strand);
(2) preparing nucleic acids as primers each having one of the plural base sequences to be detected, immobilizing the respective primers independently in separate regions on a substrate, and preparing a primer array in which the respective base sequences to be detected are distributed in the primer-immobilized regions;
(3) preparing a nucleic acid having a sequence complementary to a partial and sequential base sequence within the region between a 3'-end of the A-strand and the base sequence to be detected which is located nearest the 3'-end as a primer for elongating the B-strand, and preparing a nucleic acid having a partial and sequential base sequence within the region between a 5'-end of the A-strand and the base sequence to be detected which is located nearest the 5'-end as a primer for elongating the A-strand;
(4) performing PCR reactions using the A-strand and the B-strand as templates, and using the primers immobilized on the substrate, the primer for elongating the A-strand, and the primer for elongating the B-strand, and nucleotide monomers with a part or all of at least one group of the nucleotide monomer being labeled; and
(5) detecting a nucleic acid corresponding to the A-strand which has been elongated and amplified from a primer binding to the substrate via the label incorporated in the nucleic acid.

3. A method of detecting a nucleic acid according to claim 1 or 2,
wherein in step (1) plural single-stranded nucleic acids each having a partial and sequential base sequence to be detected (A-strand group: Al-strand to An-strand: no 2) and a group of single-stranded nucleic acids each having a base sequence complementary to a base sequence of each strand of the A-strand group (B-strand group: B1-strand to Bn-strand: n ≥ 2) are prepared;
in step (3) nucleic acids each having a sequence complementary to a partial and sequential base sequence within the region between a 3'-end of each strand of the A-strand group and the base sequence to be detected which is located nearest the 3'-end as primers for elongating the B-strands (PB-strand group: PB1-strand to PBn-strand: n ≥ 2) are prepared;
in step (4) said PCR reactions using each strand of the A-strand group and each corresponding strand of B-strand group as templates, and using the primers immobilized on the substrate, and the plural primers for elongating the B-strands of the PB-strand group are performed; and
in step (5) a hybridized product of a nucleic acid corresponding to the A-strand group which has been elongated and amplified as a result of the PCR reactions and bound to the substrate and a nucleic acid corresponding to the B-strand group which has been elongated and amplified and has not bound to the substrate is formed.

4. A method of detecting a nucleic acid according to claim 3,
wherein in step (3) additionally nucleic acids each having a partial and sequential base sequence within the region between a 5'-end of each strand of the A-strand group and the base sequence to be detected which is located nearest the 5'-end as primers for elongating the A-strands (PA-strand group: PA1-strand to PAn-strand: n ≥ 2) are prepared, and
in step (4) said PCR reactions using each strand of the A-strand group and each corresponding strand of the B-strand group as templates, and using the primers immobilized on the substrate, the primers for elongating the A-strands of the PA-strand group, and the primer for elongating the B-strand of the PB-strand group are performed.

5. A method of detecting a nucleic acid according to any one of claims 1 to 4, further comprising a step of washing and removing a reaction solution on the substrate after the PCR reactions.

6. A method of detecting a nucleic acid according to any one of claims 1 and 2 to 4, wherein the primer for elongating the B-strand is labeled, and the hybridized product is detected using the label.

7. A method of detecting a nucleic acid according to claim 6, wherein the label is a fluorescent dye.

8. A method of detecting a nucleic acid according to claim 7, further comprising a step of observing the fluorescent dye using a confocal fluorescent microscope for detecting the hybridized product.

9. A method of detecting a nucleic acid according to any one of claims 1 and 2 to 4, wherein the hybridized product is detected using a fluorescent dye as an intercalator or a groove binder which interacts with a double-stranded nucleic acid.

10. A method of detecting a nucleic acid according to claim 9, further comprising a step of observing the fluorescent dye using a confocal fluorescent microscope for detecting the hybridized product.

11. A method of quantitative determination of a nucleic acid based on signals detected according to any one of claims 1 to 10.

12. A method of detecting a nucleic acid according to any one of claims 2 to 5, wherein the label is a fluorescent dye.

13. A method of detecting a nucleic acid according to claim 12, further comprising a step of observing the fluorescent dye using a confocal fluorescent microscope for detecting the hybridized product.

14. A method of detecting a nucleic acid according to any one of claims 1 to 10, wherein at least the PCR reactions and nucleic acid detections are performed in a form in which the primer arrays are present in the same container.

15. A method of detecting a nucleic acid according to claim 14, wherein the respective PCR reactions and nucleic acid detections are performed while observing intermittently using the same means.

## Patentansprüche

1. Verfahren zum Nachweis einer Nukleinsäure mit den Schritten:
(1) Präparieren einer einzelsträngigen Nukleinsäure mit einer Mehrzahl von partiellen und sequenziellen, nachzuweisenden Basensequenzen (A-Strang) und einer einzelsträngigen Nukleinsäure mit einer zu einer Basensequenz des A-Strangs komplementären Basensequenz (B-Strang);
(2) Präparieren von Nukleinsäuren als Primer, wobei jede eine der mehreren nachzuweisenden Basensequenzen hat, unabhängiges Immobilisieren der entsprechenden Primer in separaten Bereichen auf einem Substrat und Präparieren einer Primeranordnung in welcher die entsprechenden nachzuweisenden Basensequenzen in den Primer-immobilisierten Bereichen verteilt sind;
(3) Präparieren einer Nukleinsäure mit einer zu einer partiellen und sequenziellen Basensequenz innerhalb des Bereichs zwischen einem 3'-Ende des A-Strangs und der nachzuweisenden Basensequenz, welche am nächsten dem 3'-Ende ist, komplementären Sequenz als ein Primer für die Verlängerung des B-Strangs, und Präparieren einer Nukleinsäure mit einer partiellen und sequenziellen Basensequenz innerhalb des Bereichs zwischen einem 5'-Ende des A-Strangs und der nachzuweisenden Basensequenz, welche am nächsten dem 5'-Ende ist als ein Primer für die Verlängerung des A-Strangs;
(4) Durchführen von PCR-Reaktionen unter Verwendung des A-Strangs und des B-Strangs als Matrizen und unterVerwendung der auf dem Substrat mobilisierten Primer, des Primers für die Verlängerung des A-Strangs und des Primers für die Verlängerung des B-Strangs;
(5) Ausbilden eines hybridisierten Produkts einer Nukleinsäure entsprechend dem A-Strang, welches als ein Ergebnis der PCR-Reaktionen verlängert und amplifiziert wurde und an das Substrat gebunden ist und einer dem B-Strang entsprechenden Nukleinsäure, welche verlängert und amplifiziert wurde und nicht an das Substrat gebunden wurde; und
(6) Nachweisen der nachzuweisenden Basensequenz durch Nachweis des hybridisierten Produkts in den entsprechenden Primer-immobilisierten Bereichen in der Anordnung.

2. Verfahren zum Nachweis einer Nukleinsäure mit den Schritten:
(1) Präparieren einer einzelsträngigen Nukleinsäure mit mehreren partiellen und sequenziellen nachzuweisenden Basensequenzen (A-Strang) und einer einzelsträngigen Nukleinsäure mit einer Basensequenz komplementär zu einer Basensequenz des A-Strangs (B-Strang);
(2) Präparieren von Nukleinsäuren als Primer, wobei jede eine der nachzuweisenden mehreren Basensequenzen hat, unabhängiges Immobilisieren der entsprechenden Primer in separaten Bereichen auf einem Substrat und Präparieren einer Primeranordnung, in welcher die entsprechenden nachzuweisenden Basensequenzen in den Primer-immobilisierten Bereichen verteilt sind;
(3) Präparieren einer Nukleinsäure mit einer Sequenz komplementär zu einer partiellen und sequenziellen Basensequenz innerhalb des Bereichs zwischen einem 3'-Ende des A-Strangs und der nachzuweisenden Basensequenz, welche am nächsten dem 3'-Ende ist als ein Primer für die Verlängerung des B-Strangs, und Präparieren einer Nukleinsäure mit einer partiellen und sequenziellen Basensequenz innerhalb des Bereichs zwischen einem 5'-Ende des A-Strangs und der nachzuweisenden Basensequenz, welche am nächsten dem 5'-Ende ist, als ein Primer für die Verlängerung des A-Strangs;
(4) Durchführen von PCR-Reaktionen unter Verwendung des A-Strangs und des B-Strangs als Matrizen und unter Verwendung der auf dem Substrat immobilisierten Primer, des Primers für die Verlängerung des A-Strangs und des Primers für die Verlängerung des B-Strangs und Nukleotidmonomeren, wobei ein Teil oder alle wenigstens einer Gruppe der Nukleotidmonomere markiert ist; und
(5) Nachweis einer dem A-Strang entsprechenden Nukleinsäure, welche verlängert und amplifiziert wurde von einem Primer, der an das Substrat bindet über die in die Nukleinsäure aufgenommene Markierung.

3. Verfahren zum Nachweis einer Nukleinsäure nach Anspruch 1 oder 2,
wobei im Schritt (1) mehrere einzelsträngige Nukleinsäuren mit jeweils einer partiellen und sequenziellen nachzuweisenden Basensequenz (A-Stranggruppe: A1-Strang bis An-Strang: n ≥ 2) und einer Gruppe von einzelsträngigen Nukleinsäuren mit jeweils einer Basensequenz komplementär zu einer Basensequenz jedes Strangs der A-Stranggruppe (B-Stranggruppe: B1-Strang bis Bn-Strang: n ≥ 2) präpariert werden;
in Schritt (3) Nukleinsäuren jeweils mit einer Sequenz komplementär zu einer partiellen und sequenziellen Basensequenz innerhalb des Bereichs zwischen einem 3'-Ende jedes Strangs der A-Stranggruppe und der nachzuweisenden Basensequenz, welche am nächsten dem 3'-Ende ist als Primer für die Verlängerung der B-Stränge (PB-Stranggruppe: PB1-Strang bis PBn-Strang: n ≥ 2) präpariert werden;
in Schritt (4) die PCR-Reaktionen die jeden Strang der A-Stranggruppe und jeden entsprechenden Strang der B-Stranggruppe als Matrizen verwenden und die auf dem Substrat immobilisierten Primer, und die mehreren Primer für die Verlängerung der B-Stränge der PB-Stranggruppe verwenden, durchgeführt werden; und
in Schritt (5) ein hybridisiertes Produkt einer Nukleinsäure entsprechend der A-Stranggruppe, welche als ein Ergebnis der PCR-Reaktionen verlängert und amplifiziert wurde und an das Substrat gebunden ist, und einer zu der B-Stranggruppe entsprechenden Nukleinsäure, welche verlängert und amplifiziert wurde und nicht an das Substrat gebunden ist, ausgebildet wird.

4. Verfahren zum Nachweis einer Nukleinsäure nach Anspruch 3,
wobei in Schritt (3) zusätzliche Nukleinsäuren jeweils mit einer partiellen und sequenziellen Basensequenz innerhalb des Bereichs zwischen einer 5'-Ende jedes Strangs der A-Stranggruppe und der nachzuweisenden Basensequenz, welche am nächsten dem 5'-Ende ist als Primer für die Verlängerung der A-Stränge (PA-Stranggruppe: PA1-Strang bis PAn-Strang: n ≥ 2) präpariert werden, und
in Schritt (4) die PCR-Reaktionen, die jeden Strang der A-Stranggruppe und jeden entsprechenden Strang der B-Stranggruppe als Matrizen verwenden und die auf dem Substrat immobilisierten Primer, die Primer für die Verwendung des A-Strangs der PA-Stranggruppe und die Primer für die Verlängerung des B-Strangs der PB-Stranggruppe verwendet werden, durchgeführt werden.

5. Verfahren zum Nachweis einer Nukleinsäure nach einem der Ansprüche 1 bis 4, ferner mit einem Schritt des Waschens und Entfernens einer Reaktionslösung auf dem Substrat nach den PCR-Reaktionen.

6. Verfahren zum Nachweis einer Nukleinsäure nach einem der Ansprüche 1 und 2 bis 4, wobei der Primer für die Verlängerung des B-Strangs markiert ist und das hybridisierte Produkt unter Verwendung der Markierung nachgewiesen wird.

7. Verfahren zum Nachweis einer Nukleinsäure nach Anspruch 6, wobei die Markierung ein Fluoreszenzfarbstoff ist.

8. Verfahren zum Nachweis einer Nukleinsäure nach Anspruch 7, ferner mit einem Schritt der Beobachtung des Fluoreszenzfarbstoffs unter Verwendung eines konfokalen Fluoreszenzmikroskops für den Nachweis des hybridisierten Produkts.

9. Verfahren zum Nachweis einer Nukleinsäure nach einem der Ansprüche 1 und 2 bis 4, wobei das hybridisierte Produkt unter Verwendung eines Fluoreszenzfarbstoffs als ein Interkalator oder ein Groove-Binder, welcher mit einer doppelsträngigen Nukleinsäure interagiert, nachgewiesen wird.

10. Verfahren zum Nachweis einer Nukleinsäure nach Anspruch 9, ferner mit einem Schritt des Beobachtens des Fluoreszenzfarbstoffs unter Verwendung eines konfokalen Fluoreszenzmikroskops für den Nachweis des hybridisierten Produkts.

11. Verfahren für die quantitative Bestimmung einer Nukleinsäure basierend auf Signalen nachgewiesen gemäß einem der Ansprüche 1 bis 10.

12. Verfahren zum Nachweis einer Nukleinsäure gemäß einem der Ansprüche 2 bis 5, wobei die Markierung ein Fluoreszenzfarbstoff ist.

13. Verfahren zum Nachweis einer Nukleinsäure nach Anspruch 12, ferner mit einem Schritt, des Beobachtens des Fluoreszenzfarbstoffs unter Verwendung eines konfokalen Fluoreszenzmikroskops für den Nachweis des hybridisierten Produkts.

14. Verfahren zum Nachweis einer Nukleinsäure gemäß einem der Ansprüche 1 bis 10, wobei wenigstens die PCR-Reaktionen und die Nukleinsäurenachweise in einer Form durchgeführt werden, in welcher die Primeranordnungen in demselben Behälter vorhanden sind.

15. Verfahren zum Nachweis einer Nukleinsäure gemäß Anspruch 14, wobei die entsprechenden PCR-Reaktionen und Nukleinsäurenachweise durchgeführt werden, während sie periodisch unter Verwendung der gleichen Einrichtung beobachtet werden.

## Revendications

1. Méthode pour la détection d'un acide nucléique, comprenant les étapes consistant :
(1) à préparer un acide nucléique monocaténaire ayant une pluralité de séquences de bases partielles et séquentielles à détecter (brin A) et un acide nucléique monocaténaire ayant une séquence de bases complémentaire d'une séquence de bases du brin A (brin B) ;
(2) à préparer des acides nucléiques, comme amorces, chacun comprenant une séquence de la pluralité de séquences de bases à détecter, à immobiliser les amorces respectives indépendamment dans des régions séparées sur un substrat, et à préparer un réseau d'amorces dans lequel les séquences de bases respectives à détecter sont distribuées dans les régions portant les amorces immobilisées ;
(3) à préparer un acide nucléique ayant une séquence complémentaire d'une séquence de bases partielle et séquentielle dans la région entre une extrémité 3' du brin A et la séquence de bases à détecter qui est située le plus près de l'extrémité 3' comme amorce pour l'extension du brin B, et à préparer un acide nucléique ayant une séquence de bases partielle et séquentielle dans la région entre une extrémité 5' du brin A et la séquence de bases à détecter qui est située le plus près de l'extrémité 5' comme amorce pour l'extension du brin A ;
(4) à conduire des réactions PCR en utilisant le brin A et le brin B comme matrices, et à utiliser les amorces immobilisées sur le substrat, l'amorce pour l'extension du brin A et l'amorce pour l'extension du brin B ;
(5) à former un produit hybridé d'un acide nucléique correspondant au brin A qui a subi une extension et une amplification en résultat des réactions PCR et qui a été lié au substrat et d'un acide nucléique correspondant au brin B qui a subi une extension et une amplification et qui n'a pas été lié au substrat ; et
(6) à détecter la séquence de bases à détecter en détectant le produit hybridé dans les régions portant les amorces immobilisées respectives dans le réseau.

2. Méthode de détection d'un acide nucléique, comprenant les étapes consistant :
(1) à préparer un acide nucléique monocaténaire ayant une pluralité de séquences de bases partielles et séquentielles à détecter (brin A) et un acide nucléique monocaténaire ayant une séquence de bases complémentaire d'une séquence de bases du brin A (brin B) ;
(2) à préparer des acides nucléiques, comme amorces, chacun comprenant une séquence de la pluralité de séquences de bases à détecter, à immobiliser les amorces respectives indépendamment dans des régions séparées sur un substrat, et à préparer un réseau d'amorces dans lequel les séquences de bases respectives à détecter sont distribuées dans les régions portant les amorces immobilisées ;
(3) à préparer un acide nucléique ayant une séquence complémentaire d'une séquence de bases partielle et séquentielle dans la région entre une extrémité 3' du brin A et la séquence de bases à détecter qui est située le plus près de l'extrémité 3' comme amorce pour l'extension du brin B, et à préparer un acide nucléique ayant une séquence de bases partielle et séquentielle dans la région entre une extrémité 5' du brin A et la séquence de bases à détecter qui est située le plus près de l'extrémité 5' comme amorce pour l'extension du brin A ;
(4) à conduire des réactions PCR en utilisant le brin A et le brin B comme matrices, et utiliser les amorces immobilisées sur le substrat, l'amorce pour l'extension du brin A, et l'amorce pour l'extension du brin B, et des monomères nucléotidiques, une partie ou la totalité d'au moins un groupe de monomères nucléotidiques étant marquée ; et
(5) à détecter un acide nucléique correspondant au brin A qui a subi une extension et une amplification à partir d'une amorce se liant au substrat par l'intermédiaire du marqueur incorporé à l'acide nucléique.

3. Méthode de détection d'un acide nucléique suivant la revendication 1 ou 2,
dans laquelle, dans l'étape (1), une pluralité d'acides nucléiques monocaténaires ayant chacun une séquence de bases partielle et séquentielle à détecter (groupe de brins A : brin A1 à brin An : n ≥ 2) et un groupe d'acides nucléiques monocaténaires ayant chacun une séquence de bases complémentaire d'une séquence de bases de chaque brin du groupe de brins A (groupe de brins B : brin B1 à brin Bn : n ≥ 2) sont préparés ;
dans l'étape (3), des acides nucléiques ayant chacun une séquence complémentaire d'une séquence de bases partielle et séquentielle dans la région entre une extrémité 3' de chaque brin du groupe de brins A et la séquence de bases à détecter qui est située le plus près de l'extrémité 3' comme amorces pour l'extension du brin B (groupe de brins PB : brin PB1 à brin PBn : n ≥ 2) sont préparés ;
dans l'étape (4), lesdites réactions PCR utilisant chaque brin du groupe de brins A et chaque brin correspondant du groupe de brins B comme matrices et utilisant les amorces immobilisées sur le substrat, et la pluralité d'amorces pour l'extension des brins B du groupe de brins PB, sont conduites ; et
dans l'étape (5), un produit hybridé d'un acide nucléique correspondant au groupe de brins A qui a subi une extension et une amplification en résultat des réactions PCR et qui a été lié au substrat et d'un acide nucléique correspondant au groupe de brins qui a subi une extension et une amplification et qui n'a pas été lié au substrat est formé.

4. Méthode de détection d'un acide nucléique suivant la revendication 3,
dans laquelle, dans l'étape (3), en outre, des acides nucléiques ayant chacun une séquence de bases partielle et séquentielle dans la région entre une extrémité 5' de chaque brin du groupe de brins A et la séquence de bases à détecter qui est située le plus près de l'extrémité 5' comme amorces pour l'extension des brins A (groupe de brins PA : brin PA1 à brin PAn : n ≥ 2) sont préparés ; et
dans l'étape (4), lesdites réactions PCR utilisant chaque brin du groupe de brins A et chaque brin correspondant du groupe de brins B comme matrices, et utilisant les amorces immobilisées sur le substrat, les amorces pour l'extension des brins A du groupe de brins PA, et l'amorce pour l'extension du brin B du groupe de brins PB sont conduites.

5. Méthode de détection d'un acide nucléique suivant l'une quelconque des revendications 1 à 4, comprenant en outre une étape de lavage et d'élimination d'une solution réactionnelle sur le substrat après les réactions PCR.

6. Méthode de détection d'un acide nucléique suivant l'une quelconque des revendications 1 et 2 à 4, dans laquelle l'amorce pour l'extension du brin B est marquée, et le produit hybridé est détecté en utilisant le marqueur.

7. Méthode de détection d'un acide nucléique suivant la revendication 6, dans laquelle le marqueur est un colorant fluorescent.

8. Méthode de détection d'un acide nucléique suivant la revendication 7, comprenant en outre une étape d'observation du colorant fluorescent en utilisant un microscope fluorescent confocal pour la détection du produit hybridé.

9. Méthode de détection d'un acide nucléique suivant l'une quelconque des revendications 1 et 2 à 4, dans laquelle le produit hybridé est détecté en utilisant un colorant fluorescent comme agent d'intercalation ou agent de liaison au sillon, qui interagit avec un acide nucléique bicaténaire.

10. Méthode de détection d'un acide nucléique suivant la revendication 9, comprenant en outre une étape d'observation du colorant fluorescent en utilisant un microscope fluorescent confocal pour la détection du produit hybridé.

11. Méthode de détermination quantitative d'un acide nucléique sur la base des signaux détectés suivant l'une quelconque des revendications 1 à 10.

12. Méthode de détection d'un acide nucléique suivant l'une quelconque des revendications 2 à 5, dans laquelle le marqueur est un colorant fluorescent.

13. Méthode de détection d'un acide nucléique suivant la revendication 12, comprenant en outre une étape d'observation du colorant fluorescent en utilisant un microscope fluorescent confocal pour la détection du produit hybridé.

14. Méthode de détection d'un acide nucléique suivant l'une quelconque des revendications 1 à 10, dans laquelle au moins les réactions PCR et les détections d'acides nucléiques sont effectuées sous une forme dans laquelle les réseaux d'amorces sont présents dans le même récipient.

15. Méthode de détection d'un acide nucléique suivant la revendication 14, dans laquelle les réactions PCR et détections d'acides nucléiques respectives sont effectuées tout en effectuant des observations de manière intermittente en utilisant le même moyen.
